# EUROPEAN PATENT APPLICATION

(11) **EP 3 184 515 A1**
(43) Date of publication of application: **28.06.2017**
(21) Application number: 15201920.4
(22) Date of filing: 22.12.2015
(51) Int. Cl.: C07D 265/36

(54) **PROCESS FOR MANUFACTURING 1,4-BENZOXAZINONES**

(71) Applicant: BASF SE, 67056 Ludwigshafen am Rhein (DE)
(72) Inventor: WOLF, Bernd, 67150 Niederkirchen (DE); GOETZ, Roland, 68809 Neulussheim (DE); KAPPE, Oliver C., 8010 Graz (AT); CANTILLO, David, 8010 Graz (AT)
(74) Representative: BASF IP Association

(57) **Abstract**

The present invention relates to a continuous flow synthesis for manufacturing 1,4-benzoxazinones of formula (1) 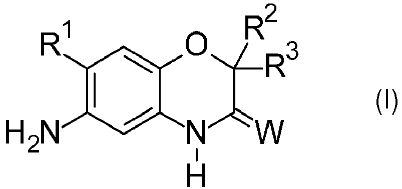 wherein
R¹
is H or halogen;
R²
is halogen;
R³
is H or halogen; and
W
is O or S;

via a nitration-hydrogenation-cyclization sequence.

## Description

The present invention relates to a continuous flow synthesis for manufacturing 1,4-benzoxazinones of formula (I) 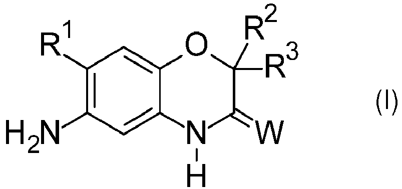 wherein
- R¹: is H or halogen;
- R²: is halogen;
- R³: is H or halogen; and
- W: is O or S;
via a nitration-hydrogenation-cyclization sequence.

1,4-benzoxazinones of formula (I) are valuable intermediates, for example for preparing herbicidal ingredients.

WO 13/92856 describes a process for preparing 1,4-benzoxazinones of formula (I), wherein first a nitro compound is reacted with a reducing agent followed by reacting the resulting amino compound with an acid resulting in 86% of the desired 1,4-benzoxazinone.

WO 15/158518 describes a process for preparing 1,4-benzoxazinones of formula (I) by first reacting a nitro compound with a reducing agent followed by reacting the resulting amino compound with an acid, wherein the amino compound is added to the acid resulting in 95% of the desired 1,4-benzoxazinone.

Using aryloxyacetamides of formula (IV) as starting material the desired 1,4-benzoxazinones of formula (I) can be prepared in a three-step sequence:

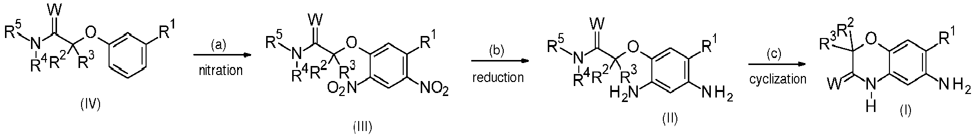

In the first step (a) the aryloxyacetamide of formula (IV) is dinitrated using a suitable nitrating mixture to give respective nitro compounds of formula (III). Then, the nitro groups are selectively reduced and the resulting amino compound of formula (II) is then cyclized under acidic conditions to give 1,4-benzoxazinones of formula (I).

This apparently straightforward procedure presents, however, significant complications especially when carried out on large scale. The first intermediate in the sequence, the nitro compound of formula (III), is a potentially explosive material as indicated by the Könen tube test. Thus, isolation and/or storage of the dinitro intermediate is not desired. Moreover, the nitro compound of formula (III) is an unstable, air and light sensitive compound, particularly problematic when stored in solution.

Due to these limitations, it was an object of the invention to provide a one-pot sequential process which directly transforms the aryloxyacetamide of formula (IV) into 1,4-benzoxazinones of formula (I).

Apart from the above mentioned issues related to the stability of the nitro- and amino-compounds (III) and (II), nitration and hydrogenation reactions on their own constitute inherently hazardous transformations when performed on a large scale. Nitration reactions are generally highly exothermic and constitute one the most hazardous industrial processes. Safety risks are even more significant when the produced nitro compound has explosive properties as in the case of nitro-compound of formula (III).
Against that background, continuous flow processing has been shown as a safe alternative for the preparation of nitro compounds, and in the past years a number of continuous flow nitrations have been reported in the literature (e.g. Kulkarni, A. A. Beilstein J. Org. Chem. 2014, 10, 405-424). The enhanced heat transfer experienced in microfluidic devices can control the exotherm generated during nitration reactions. Therefore these hazardous processes can be carried out in a safe, controlled, and scalable manner using flow devices (e.g. Gutmann, B., Cantillo, D., Kappe, C. O. Angew. Chem. Int. Ed. DOI: 10.1002/anie.201409318). A typical experimental setup for continuous nitration of aromatic compounds consist of two feeds, containing the substrate and the nitrating agent, respectively. The two feeds are mixed in a micromixing device under thermostated conditions, followed by a residence time unit.

Catalytic hydrogenations using batch conditions are also inherently hazardous as pressurized hydrogen gas can cause explosions. Thus, pressure resistant autoclave reactors and special safety precautions are typically required. Continuous flow hydrogenation is a common technique in modern organic synthesis (e.g. Irfan, M.; Glasnov, T. N.; Kappe, C. O. ChemSus-Chem, 2011, 4, 300-316.), and specifically designed reactors for catalytic hydrogenations on laboratory scale are commercially available. In addition to the safe, on demand generation of hydrogen gas characteristic of this type of devices, pre-packed catalyst cartridges are employed, thus avoiding the handling of often pyrophoric metal catalysts.

Surprisingly it has been found that 1,4-benzoxazinones of formula (I) can be prepared by a fully integrated continuous three-step nitration-hydrogenation-cyclization sequence.

It is therefore one object of the present invention to provide a process for manufacturing 1,4-benzoxazinones of formula (I), 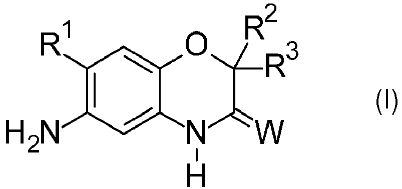 wherein
- R¹: is H or halogen;
- R2: is halogen;
- R³: is H or halogen; and
- W: is O or S
characterized in that in a fully integrated continuous process the following steps (a) to (c) are performed:
step (a): dinitrating the aryloxyacetamide of formula (IV) 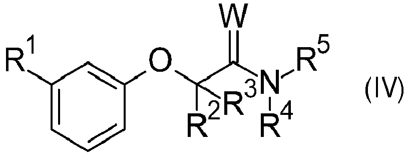 wherein
   - R¹: is H or halogen;
   - R²: is halogen;
   - R³: is H or halogen;
   and
   - R⁴, R⁵: are independently of each other C₁-C₆-alkyl, C₂-C₆-alkenyl, C₂-C₆-alkynyl, C₁-C₆-haloalkyl, C₁-C₆-cyanoalkyl, C₁-C₆-nitroalkyl, C₁-C₆-hydroxyalkyl, C₁-C₆-alkoxy-C₁-C₆-alkyl, amino-C₁-C₆-alkyl, (C₁-C₆-alkyl)amino-C₁-C₆-alkyl, di(C₁-C₆-alkyl)amino-C₁-C₆-alkyl, C₃-C₆-cycloalkyl, phenyl or benzyl, wherein the phenyl and the benzyl ring are independently of one another unsubstituted or substituted with1 to 5 substituents selected from the group consisting of halogen, C₁-C₆-alkyl or C₁-C₆-alkoxy; or
   - R⁵ and R⁶: together with the N atom which they are attached to, represent a saturated or aromatic 3- to 6-membered ring, optionally containing 1 to 3 additional heteroatoms from the group O, S and N, with the ring optionally being substituted with 1 to 3 C₁-C₆-alkyl substituents; and W isOorS;
followed by step (b): reduction of the nitro compounds of formula (III) resulting from step (a): 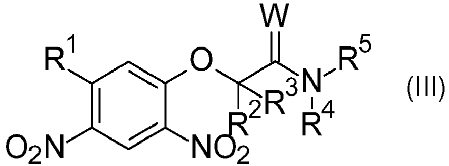 wherein R¹, R², R³, R⁴, R⁵ and W are defined as above;
followed by step (c): cyclization of the amino compounds of formula (II) resulting from step (b): 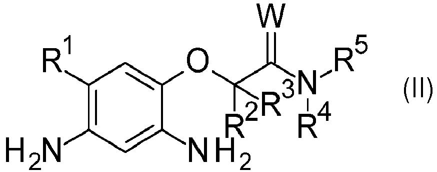 wherein R¹, R², R³, R⁴, R⁵ and W are defined as above;
   under acidic conditions to give 1,4-benzoxazinones of formula (I).

The organic moieties mentioned in the definition of the variables according to the present invention, e.g. R¹ to R⁵ are - like the term halogen - collective terms for individual enumerations of the individual group members.

The term halogen denotes in each case fluorine, chlorine, bromine or iodine. All hydrocarbon chains, i.e. all alkyl, can be straight-chain or branched, the prefix Cₙ-Cₘ denoting in each case the possible number of carbon atoms in the group.

Examples of such meanings are:
- C₁-C₄-alkyl: for example CH₃, C₂H₅, n-propyl, CH(CH₃)₂ n-butyl, CH(CH₃)-C₂H₅, CH₂-CH(CH₃)₂ and C(CH₃)₃;
- C₁-C₆-alkyl and also the C₁-C₆-alkyl moieties of C₁-C₆-cyanoalkyl, C₁-C₆-nitroalkyl, C₁-C₆-hydroxyalkyl, C₁-C₆-alkyoxy-C₁-C₆-alkyl, amino-C₁-C₆-alkyl, (C₁-C₆-alkyl)amino-C₁-C₆-alkyl and di(C₁-C₆-alkyl)amino-C₁-C₆-alkyl: C₁-C₄-alkyl as mentioned above, and also, for example, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl, preferably methyl, ethyl, n-propyl, 1-methylethyl, n-butyl, 1,1-dimethylethyl, n-pentyl or n-hexyl;
- C₁-C₄-haloalkyl: a C₁-C₄-alkyl radical as mentioned above which is partially or fully substituted by fluorine, chlorine, bromine and/or iodine, for example, chloromethyl, dichloromethyl, trichloromethyl, fluoromethyl, difluoromethyl, trifluoromethyl, chlorofluoromethyl, dichlorofluoromethyl, chlorodifluoromethyl, bromomethyl, iodomethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3-pentafluoropropyl, heptafluoropropyl, a C₁-C₃-haloalkyl radical as mentioned above, and also, for example, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl, nonafluorobutyl, 1,1,2,2,-tetrafluoroethyl and 1-trifluoromethyl-1,2,2,2-tetrafluoroethyl;

- C₁-C₆-haloalkyl: C₁-C₄-haloalkyl as mentioned above, and also, for example, 5-fluoropentyl, 5-chloropentyl, 5-bromopentyl, 5-iodopentyl, undecafluoropentyl, 6-fluorohexyl, 6-chlorohexyl, 6-bromohexyl, 6-iodohexyl and dodecafluorohexyl;
- C₃-C₆-cycloalkyl: monocyclic saturated hydrocarbons having 3 to 6 ring members, such as cyclopropyl, cyclobutyl, cyclopentyl and cyclohexyl;
- C₃-C₆-alkenyl: for example 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl;
- C₂-C₆-alkenyl: C₃-C₆-alkenyl as mentioned above, and also ethenyl;
- C₃-C₆-alkynyl: for example 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl;
- C₂-C₆-alkynyl: C₃-C₆-alkynyl as mentioned above and also ethynyl;
- C₁-C₄-alkoxy: for example methoxy, ethoxy, propoxy, 1-methylethoxy butoxy, 1-methylpropoxy, 2-methylpropoxy and 1,1-dimethylethoxy;
- C₁-C₆-alkoxy: C₁-C₄-alkoxy as mentioned above, and also, for example, pentoxy, 1-methylbutoxy, 2-methylbutoxy, 3-methoxylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy.
- (C₁-C₄-alkyl)amino: for example methylamino, ethylamino, propylamino, 1-methylethylamino, butylamino, 1-methylpropylamino, 2-methylpropylamino or 1,1-dimethylethylamino;
- (C₁-C₆-alkyl)amino and also the (C₁-C₆-alkyl)amino moieties of (C₁-C₆-alkyl)amino-C₁-C₆-alkyl: (C₁-C₄-alkylamino) as mentioned above, and also, for example, pentylamino, 1-methylbutylamino, 2-methylbutylamino, 3-methylbutylamino, 2,2-dimethylpropylamino, 1-ethyl-propylamino, hexylamino, 1,1-dimethylpropylamino, 1,2-dimethylpropylamino, 1-methylpentylamino, 2-methylpentylamino, 3-methylpentylamino, 4-methylpentylamino, 1,1-dimethylbutylamino, 1,2-dimethylbutylamino, 1,3-dimethylbutylamino, 2,2-dimethylbutylamino, 2,3-dimethylbutyl-amino 3,3-dimethylbutylamino, 1-ethylbutylamino, 2-ethylbutylamino, 1,1,2-trimethylpropylamino, 1,2,2-trimethyl-propylamino, 1-ethyl-1-methylpropylamino or 1-ethyl-2-methylpropylamino;
- di(C₁-C₄-alkyl)amino: for example N,N-dimethylamino, N,N-diethylamino, N,N-di(1-methylethyl)amino, N,N-dipropylamino, N,N-dibutylamino, N,N-di(1-methylpropyl)amino, N,N-di(2-methylpropyl)amino, N,N-di(1,1-dimethylethyl)amino, N-ethyl-N-methylamino, N-methyl-N-propylamino, N-methyl-N-(1-methylethyl)amino, N-butyl-N-methylamino, N-methyl-N-(1-methylpropyl)amino, N-methyl-N-(2-methylpropyl)amino, N-(1,1-dimethylethyl)-N-methylamino, N-ethyl-N-propylamino, N-ethyl-N-(1-methylethyl)amino, N-butyl-N-ethylamino, N-ethyl-N-(1-methylpropyl)amino, N-ethyl-N-(2-methylpropyl)amino, N-ethyl-N-(1,1-dimethylethyl)amino, N-(1-methylethyl)-N-propylamino, N-butyl-N-propylamino, N-(1-methylpropyl)-N-propylamino, N-(2-methylpropyl)-N-propylamino, N-(1,1-dimethylethyl)-N-propylamino, N-butyl-N-(1-methylethyl)amino, N-(1-methylethyl)-N-(1-methylpropyl)amino, N-(1-methylethyl)-N-(2-methylpropyl)amino, N-(1,1-dimethylethyl)-N-(1-methylethyl)amino, N-butyl-N-(1-methylpropyl)amino, N-butyl-N-(2-methylpropyl)amino, N-butyl-N-(1,1-dimethylethyl)amino, N-(1-methylpropyl)-N-(2-methylpropyl)amino, N-(1,1-dimethylethyl)-N-(1-methylpropyl)amino or N-(1,1-dimethylethyl)-N-(2-methylpropyl)amino;
- di(C₁-C₆-alkyl)amino and also the di(C₁-C₆-alkyl)amino moieties of di(C₁-C₆-alkyl)amino-C₁-C₆-alkyl: di(C₁-C₄-alkyl)amino as mentioned above, and also, for example, N-methyl-N-pentylamino, N-methyl-N-(1-methylbutyl)amino, N-methyl-N-(2-methylbutyl)amino, N-methyl-N-(3-methylbutyl)amino, N-methyl-N-(2,2-dimethylpropyl)amino, N-methyl-N-(1-ethylpropyl)amino, N-methyl-N-hexylamino, N-methyl-N-(1,1-dimethylpropyl)amino, N-methyl-N-(1,2-dimethylpropyl)amino, N-methyl-N-(1-methylpentyl)amino, N-methyl-N-(2-methylpentyl)amino, N-methyl-N-(3-methylpentyl)amino, N-methyl-N-(4-methylpentyl)amino, N-methyl-N-(1,1-dimethylbutyl)amino, N-methyl-N-(1,2-dimethylbutyl)amino, N-methyl-N-(1,3-dimethylbutyl)amino, N-methyl-N-(2,2-dimethylbutyl)amino, N-methyl-N-(2,3-dimethylbutyl)amino, N-methyl-N-(3,3-dimethylbutyl)amino, N-methyl-N- (1-ethylbutyl)amino, N-methyl-N-(2-ethylbutyl)amino, N-methyl-N-(1,1,2-trimethylpropyl)amino, N-methyl-N- (1,2,2-trimethylpropyl)amino, N-methyl-N-(1-ethyl-1-methylpropyl)amino, N-methyl-N- (1-ethyl-2-methylpropyl)amino, N-ethyl-N-pentylamino, N-ethyl-N-(1-methylbutyl)amino, N-ethyl-N-(2-methylbutyl)amino, N-ethyl-N-(3-methylbutyl)amino, N-ethyl-N-(2,2-dimethylpropyl)amino, N-ethyl-N-(1-ethylpropyl)amino, N-ethyl-N-hexylamino, N-ethyl-N-(1,1-dimethylpropyl)amino, N-ethyl-N-(1,2-dimethylpropyl)amino, N-ethyl-N-(1-methylpentyl)amino, N-ethyl-N-(2-methylpentyl)amino, N-ethyl-N-(3-methylpentyl)amino, N-ethyl-N-(4-methylpentyl)amino, N-ethyl-N-(1,1-dimethylbutyl)amino, N-ethyl-N-(1,2-dimethylbutyl)amino, N-ethyl-N-(1,3-dimethylbutyl)amino, N-ethyl-N-(2,2-dimethylbutyl)amino, N-ethyl-N-(2,3-dimethylbutyl)amino, N-ethyl-N-(3,3-dimethylbutyl)amino, N-ethyl-N-(1-ethylbutyl)amino, N-ethyl-N-(2-ethylbutyl)amino, N-ethyl-N-(1,1,2-trimethylpropyl)amino, N-ethyl-N-(1,2,2-trimethylpropyl)amino, N-ethyl-N-(1-ethyl-1-methylpropyl)amino, N-ethyl-N-(1-ethyl-2-methylpropyl)amino, N-propyl-N-pentylamino, N-butyl-N-pentylamino, N,N-dipentylamino, N-propyl-N-hexylamino, N-butyl-N-hexylamino, N-pentyl-N-hexylamino or N,N-dihexylamino;
- saturated or aromatic 3- to 6-membered ring optionally containing 1 to 3 additional heteroatoms selected from the group O, S and N:
   a monocyclic, saturated or aromatic cycle having three to six ring members which comprises apart from one nitrogen atom and carbon atoms optionally additionally one to three heteroatoms selected from the group O, S and N, for example:
      1-aziridinyl, 1-azetidinyl; 1-pyrrolidinyl, 2-isothiazolidinyl, 2-isothiazolidinyl, 1-pyrazolidinyl, 3-oxazolidinyl, 3-thiazolidinyl, 1-imidazolidinyl, 1,2,4-triazolidin-1-yl, 1,2,4-oxadiazolidin-2-yl, 1,2,4-oxadiazolidin-4-yl, 1,2,4-thiadiazolidin-2-yl, 1,2,4-thiadiazolidin-4-yl; 1-pyrrolyl, 1-pyrazolyl, 1-imidazolyl, 1,2,3-triazol-1-yl, 1,2,4-triazol-1-yl, 1-tetrazolyl; 1-piperidinyl, 1-hexahydropyridazinyl, 1-hexahydropyrimidinyl, 1-piperazinyl, 1,3,5-hexahydrotriazin-1-yl, 1,2,4-hexahydrotriazin-1-yl, tetrahydro-1,3-oxazin-1-yl, 1-morpholinyl.

The preferred embodiments of the invention mentioned herein below have to be understood as being preferred either independently from each other or in combination with one another.

According to a preferred embodiment of the invention preference is also given to the preparation of those 1,4-benzoxazinones of formula (I), wherein the variables, either independently of one another or in combination with one another, have the following meanings:
- R¹: is preferably H or F; particularly preferred H; is also preferably halogen, particularly preferred F or Cl, especially preferred F;
- R²: is preferably Cl or F, particularly preferred F;
- R³: is preferably H, Cl or F, particularly preferred H or F, especially preferred H; is also preferably halogen, particularly preferred F or Cl, especially preferred F; and
- W: is preferably O, is also preferably S.

Preference is also given to the preparation of those 1,4-benzoxazinones of formula (I), wherein R¹ and R³ are halogen and R² is F.

Preference is also given to the preparation of those 1,4-benzoxazinones of formula (I), wherein R⁴ and R⁵ independently of each other are C₁-C₆-alkyl.

Preference is also given to the preparation of those 1,4-benzoxazinones of formula (I), wherein R¹ and R³ are halogen, R² is F, and R⁴ and R⁵ independently of each other are C₁-C₆-alkyl.

Particular preference is also given to the preparation of benzoxazinones of formula (Ia), which correspond to 1,4-benzoxazinones of formula (I) wherein R³ is F:

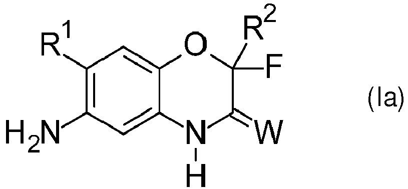

Particular preference is also given to the preparation of benzoxazinones of formula (Ia.1), which correspond to 1,4-benzoxazinones of formula (I) wherein R² and R³ are F:

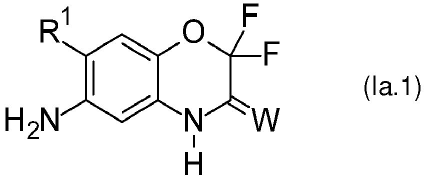

Particular preference is also given to the preparation of the benzoxazinone of formula (Ia.1.1), which corresponds to benzoxazinones of formula (I) wherein R¹, R² and R³ are F, and W is O:

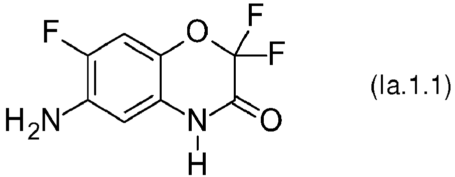

With respect to the variables within the amino compound of formula (II), the nitro compounds of formula (III) and the aryloxyacetamides of formula (IV), the particularly preferred embodiments correspond, either independently of one another or in combination with one another, to those of the variables of R¹, R², R³ and W of formula (I), or have, either independently of one another or in combination with one another, the following meanings:
R⁴ and R⁵ preferably are independently of each other C₁-C₆-alkyl, C₁-C₆-cyanoalkyl, C₁-C₆-hydroxyalkyl, C₁-C₆-alkoxy-C₁-C₆-alkyl, C₃-C₆-cycloalkyl, phenyl or benzyl, wherein the phenyl and the benzyl ring are independently of one another unsubstituted or substituted with 1 to 3 substituents selected from the group consisting of halogen, C₁-C₆-alkyl or C₁-C₆-alkoxy,
   or R⁴ and R⁵ together with the N atom which they are attached to, represent a saturated or aromatic 5- to 6-membered ring, optionally containing 1 additional heteroatom from the group O and N, with the ring optionally being substituted with 1 to 2 C₁-C₆-alkyl substituents;
   particularly preferred are independently of each other C₁-C₄-alkyl, C₁-C₄-hydroxyalkyl, C₁-C₆-alkoxy-C₁₋C₄-alkyl or benzyl, wherein the benzyl ring is unsubstituted or substituted by 1 to 3 substituents selected from the group consisting of halogen, C₁-C₄-alkyl or C₁-C₄-alkoxy, especially preferred the benzyl ring is unsubstituted,
   or R⁴ and R⁵ together with the N atom which they are attached to, represent a saturated 5-to 6-membered ring, optionally containing 1 additional oxygen atom, with the ring optionally being substituted with 1 to 2 C₁-C₆-alkyl substituents;
   also particularly preferred are independently of one another C₁-C₆-alkyl.

To overcome the above mentioned issues for the large scale preparation of 1,4-benzoxazinones of formula (I), a fully continuous flow protocol for the synthesis of 1,4-benzoxazinones of formula (I) has been found, in which aryloxyacetamide of formula (IV), used as starting material, is subjected to a sequential continuous flow nitration/hydrogenation/cyclization.

The uninterrupted continuous flow processing was achieved by integrating liquid-liquid membrane separation technology and the inline removal of excess of hydrogen gas using gas permeable tubing into the process.

In the first step (a) the aryloxyacetamide of formula (IV) is dinitrated using a suitable nitrating mixture to give respective nitro compounds of formula (III).

The aryloxyacetamide of formula (IV) is subjected to an initial continuous flow dinitration, preferably using 20% oleum in combination with 100% HNO₃ (2.5 equiv).
More preferably a microstructured device heated to 60 °C is used.

The explosive nitro compound of formula (III) generated is extracted from the nitrating mixture using a suitable organic solvent.
Preferably it is separated from the reaction mixture with a continuous liquid-liquid membrane separator.

The organic phase containing the nitro compound of formula (III) is then subjected to continuous hydrogenation, immediately consuming the nitro compound of formula (III) without the need for isolation or purification, thus avoiding the handling of this dangerous material.

In the second step (b) the nitro compound of formula (III) is reduced to give respective amino compounds of formula (II).

Preferably the reduction is a continuous flow hydrogenation of the nitro compound of formula (III) over a Pd/C fixed bed catalyst.
More preferably the reduction is made at 45 °C.

In the third step (c) the resulting air-sensitive amino compound of formula (II) is then directly cyclized to the desired 1,4-benzoxazinone of formula (I) via an acid-catalyzed cyclization step.
Preferably such cyclization is made at 80 °C
Also preferably the resulting solution of the amino compound of formula (II) is directly acidified and heated under flow conditions.

More preferably a tubular reactor is used.

The invention is illustrated by the following examples without being limited thereto or thereby.

### General

¹H-NMR spectra were recorded on a Bruker 300 MHz instrument. ¹³C-NMR spectra were recorded on the same instrument at 75 MHz. Chemical shifts (δ) are expressed in ppm downfield from TMS as internal standard. The letters s, d, t, q and m are used to indicate singlet, doublet, triplet, quadruplet and multiplet.

Analytical HPLC-UV (Shimadzu LC20) analysis was carried out on a C18 reversed-phase (RP) analytical column (150 × 4.6 mm, particle size 5 µm) at 37 °C using a mobile phase A (water/acetonitrile 90:10 (v/v) + 0.1 % TFA) and B (MeCN + 0.1 % TFA) at a flow rate of 1.5 mL min⁻¹. Details on the gradients applied are as follows:
Nitration mixtures:
   Mobile phase A: H₂O/MeCN 9:1 (v/v) + 0.1 vol% TFA;
   Mobile phase B: MeCN + 0.1 vol% TFA
   Flow rate 1.5 mL/min
   Gradient: linear increase from solution 20% B to 40% B in 10 min, linear increase from solution 40% B to 100% B in 4 min; hold at 100% solution B for 2 min
Hydrogenation and cyclization mixtures:
   Mobile phase A: H₂O/MeCN 9:1 (v/v) + 0.1 vol% TFA;
   Mobile phase B: MeCN + 0.1 vol% TFA
   Flow rate 1.5 mL/min
   Gradient: linear increase from solution 10% B to 50% B in 10 min, linear increase from solution 50% B to 60% B in 5 min; hold at 60% solution B for 2 min.

GC/MS monitoring was based on electron impact ionization (70 eV) using a HP/5MS column (30 m×0.250 mm×0.025 µm). After 1 min at 50°C the temperature was increased in 25°Cmin⁻¹ steps up to 300°C and kept at 300°C for 1 min. The carrier gas was helium and the flow rate 1.0 mLmin⁻¹ in constant-flow mode. All chemicals were purchased from commercial sources and were used without further purification. 2,2-difluoro-2-(3-fluoro-phenoxy)-N,N-dimethyl-acetamide was prepared according to WO 14/026893. CatCart pre-packed Pd/C catalyst cartridges (THS X1175) for the H-Cube Pro were obtained from ThalesNano. ICMPS

### Batch Nitration of 2,2-difluoro-2-(3-fluoro-phenoxy)-N,N-dimethyl-acetamide

To a vial containing fuming sulfuric acid (oleum, 20% SO₃) (1 mL) was added 100% nitric acid (2.2 or 2.5 equiv) (Figure 2) under stirring at 0 °C. Subsequently, 2,2-difluoro-2-(3-fluoro-phenoxy)-N,N-dimethyl-acetamide (2 mmol, 358 µL) was added dropwise, under vigorous stirring, over a period of 40-60 s. The vial was capped and heated at 50-60 °C. Reaction monitoring was carried out by collecting aliquots (~ 2 µL) from the crude reaction mixture, diluting with 2 mL MeCN/H₂O, and analyzing by HPLC after 5, 10, 15, 20 and 30 min reaction time.

### Sequential Continuous Flow Nitration/Liquid-Liquid Extraction:

With the aim of obtaining a continuous stream of 2,2-difluoro-2-(2,4-dinitro-5-fluoro-phenoxy)-N,N-dimethyl-acetamide in toluene ready to be introduced in the hydrogenation reactor (after inline addition of methanol) a nitration setup incorporating a membrane based liquid-liquid separator was built. The flow rates for the neat FPAA (2) substrate, nitration mixture, and toluene were set according to the values calculated for a productivity of 0.2 mmol min-1. To obtain a residence time of ca. 20 min in the thermostated bath a residence time unit (1.5 mL volume, 0.8 mm inner diameter PFA tubing) was added to the glass static mixer output. The residence time unit then entered a second water bath at 0-10 °C, where water and toluene are added simultaneously to the reaction mixture using an X-mixer.
By adding both solvents simultaneously at low temperature precipitation of the 2,2-difluoro-2-(2,4-dinitro-5-fluoro-phenoxy)-N,N-dimethyl-acetamide in the water phase and a possible nitration of toluene are avoided. For the extraction process a 4 mL volume tubing was installed. The inner diameter for the extraction tubing was increased to 1.6 mm to augment the contact surface between the toluene and water phases and enhance the extraction. A commercial membrane based liquid/liquid separator (Zaiput) was utilized to separate the organic and aqueous phases in continuous flow.
The continuous flow reactor was started. The system was run for 60 min to assure stability and steady-state concentration conditions. Then, 10 mL of the toluene phase were collected from the corresponding membrane separator output. In a separate cylinder 10 mL of the aqueous phase were additionally collected from the water output of the membrane separator. HPLC analyses of the organic and aqueous phase revealed excellent efficiency in the extraction process, with a toluene/water 2,2-difluoro-2-(2,4-dinitro-5-fluoro-phenoxy)-N,N-dimethyl-acetamide distribution coefficient of 38:1 (determined by 215 nm peak area integration) (HPLC chromatograms of the organic and aqueous phases are shown in Figure S8 in the Supporting Information). The organic phase was then evaporated under reduced pressure until all volatiles were removed. Cyclohexane was added and the pale yellow precipitate filtered off,17 yielding 92% of pure, 2,2-difluoro-2-(2,4-dinitro-5-fluoro-phenoxy)-N,N-dimethyl-acetamide.
This yield and purity is in contrast with the reported batch protocol, in which 82% yield of 2,2-difluoro-2-(2,4-dinitro-5-fluoro-phenoxy)-N,N-dimethyl-acetamide with 78% purity could be obtained.

Using the setup as described above, 2,2-difluoro-2-(3-fluoro-phenoxy)-N,N-dimethyl-acetamide (Feed A, 35.8 µL/min) and a solution of 100% HNO₃ (2.5 equiv) in oleum (20% SO₃) (Feed B, 100 µL/min) were pumped through the reactor using two syringe pumps (Syrris). The two streams were mixed using a static glass mixer (1.5 mL internal volume, Uniqsis) entered a 1.5 mL residence time unit (PFA tubing, 0.8 mm i.d. and 1.59 mm o.d.). The reaction mixture was mixed with water and toluene using a X-mixer at 0 °C, and entered a second residence time unit (4 mL volume, PFA tubing with 1.6 mm i.d. and 3.2 mm o.d) connected to a membrane liquid-liquid separator (Zaiput, 0.5 µm pore size, 42 × 15 mm). The organic phase obtained was used directly for the next step of synthesis without further purification. A 10 mL aliquot of the organic phase was collected and evaporated under reduced pressure.
The residue was precipitated with cyclohexane (20 mL) and filtered off, providing 0.97 g (92%) of 2,2-difluoro-2-(2,4-dinitro-5-fluoro-phenoxy)-N,N-dimethyl-acetamide as a pale yellow solid; mp. 67-68 °C.^{17 1}H NMR (300 MHz, CDCl₃) δ 8.81 (d, *J* = 7.5 Hz, 1H), 7.52 (d, *J* = 10.9 Hz, 1H), 3.25 (s, 3H), 3.09 (s, 3H); ¹³C NMR (75 MHz, CDCl₃) δ 159.3, 157.1, 155.7, 147.9, 124.6, 119.4, 115.7, 112.2, 111.9, 37.2.

### Continuous Flow Hydrogenation of 2,2-difluoro-2-(2,4-dinitro-5-fluoro-phenoxy)-N,N-dimethyl-acetamide

Hydrogenation reactions employing heterogeneous catalysts in fixed bed reactors exhibit several benefits using continuous-flow processing apart from the obvious safety advantages. In continuous flow hydrogenations, the hydrogen/substrate mixture is pumped through packed catalyst columns by high-pressure pumps. Due to the large interfacial areas and the short diffusion paths in the packed columns, very efficient gas-liquid-solid interactions, and thus hydrogenation, takes place.

Hydrogenation of 2,2-difluoro-2-(2,4-dinitro-5-fluoro-phenoxy)-N,N-dimethyl-acetamide under continuous flow conditions was performed in a commercial high pressure hydrogenator (H-Cube Pro, Thales Nanotechnology Inc.) enabling heterogeneous hydrogenations at temperatures up to 150 °C and 100 bar of hydrogen pressure. The reaction mixture is introduced into the reactor by a HPLC pump (max. 3 mL min-1). The system was equipped with a pre-packed, commercially available cartridge (70 mm length × 4 mm i.d., 0.8 mL volume) of the heterogeneous 10% Pd/C catalyst. In all cases, the instrument was set to "full H2" mode which corresponds to a gas flow rate of 60 mL min⁻¹.

The nitration/extraction process preceding the hydrogenation step produces a solution of 2,2-difluoro-2-(2,4-dinitro-5-fluoro-phenoxy)-N,N-dimethyl-acetamide in toluene, which then enters the H-Cube Pro hydrogenator. However, the resulting 2,2-difluoro-2-(2,4-diamino-5-fluoro-phenoxy)-N,N-dimethyl-acetamide product is not soluble in toluene and clogging of the reactor may occur during the hydrogenation if no co-solvent is added. Solubility tests indicated that 2,2-difluoro-2-(2,4-diamino-5-fluoro-phenoxy)-N,N-dimethyl-acetamide is well soluble in a toluene/methanol 2:1 mixture even at a concentration of 0.2 M. Thus, addition of methanol after the liquid/liquid extraction step is necessary.

A 0.3 M solution of 2,2-difluoro-2-(2,4-dinitro-5-fluoro-phenoxy)-N,N-dimethyl-acetamide in toluene (1.33 mL) (see above) was diluted with methanol (0.66 mL). The hydrogen level of the H-Cube Pro was set to "full H₂" mode, the reactor containing a Pd/C cartridge was pre-heated to 45 °C and the flow rate and pressure were set to according to Table 1. When the system was stable the reaction mixture was pumped through the reactor. The crude solution obtained from the reactor output was diluted with acetonitrile and analyzed by HPLC.

A set of continuous flow reductions was carried out to establish the optimal reaction conditions, in particular H₂ pressure and flow rate. A 0.2 M solution of 2,2-difluoro-2-(2,4-dinitro-5-fluoro-phenoxy)-N,N-dimethyl-acetamide in toluene/methanol 2:1 was used as starting reaction mixture in all cases, and the temperature was set to 45 °C The results (Table 1) indicated that a successful reduction can be carried out up to a flow rate of 1 mL min⁻¹. Although 2,2-difluoro-2-(2,4-dinitro-5-fluoro-phenoxy)-N,N-dimethyl-acetamide was consumed in all cases, it was not fully converted to 2,2-difluoro-2-(2,4-diamino-5-fluoro-phenoxy)-N,N-dimethyl-acetamide when a flow rate of 2 mL min⁻¹ was employed, even at a pressure of 20 bar. HPLC and GC-MS analyses of the crude reaction mixtures obtained at 1 mL min⁻¹ revealed that the desired 2,2-difluoro-2-(2,4-diamino-5-fluoro-phenoxy)-N,N-dimethyl-acetamide was obtained in very high purity.

**Table 1: Results obtained for the continuous hydrogenation of 2,2-difluoro-2-(2,4-dinitro-5-fluoro-phenoxy)-N,N-dimethyl-acetamide in the H-Cube Pro using Pd/C as catalyst.**

| Entry | Flow Rate (mL min⁻¹) | Pressure (bar) | Substrate Conversion (%)^{a} | Diamino/monoamino Selectivity (%)^{b} |
|---|---|---|---|---|
| 1 | 0.5 | 1 | > 99 | > 99 |
| 2 | 1 | 1 | > 99 | > 99 |
| 3 | 1 | 10 | > 99 | > 99 |
| 4 | 1 | 20 | > 99 | > 99 |
| 5 | 2 | 1 | > 99 | 21 |
| 6 | 2 | 10 | > 99 | 38 |
| 7 | 2 | 20 | >99 | 77 |

| | | | | |
|---|---|---|---|---|
| ^{a} HPLC peak integration area (215 nm). ^{b} GC-MS peak integration area. | | | | |

### Sequential Continuous Hydrogenation/Cyclization Process

The hydrogenation reaction mixture exiting the H-Cube Pro reactor consists of a solution of 2,2-difluoro-2-(2,4-diamino-5-fluoro-phenoxy)-N,N-dimethyl-acetamide in toluene/methanol 2:1 together with a large amount of H₂ gas. Although the presence of H₂ gas should not affect the cyclization reaction, it does have an influence in the process because the residence time cannot be easily controlled. We therefore performed an inline removal of the excess of H₂ after the reduction using gas permeable Teflon AF2400 tubing. As 2,2-difluoro-2-(2,4-diamino-5-fluoro-phenoxy)-N,N-dimethyl-acetamide is relatively unstable in the presence or air and light, we decided to optimize both degassing and cyclization simultaneously, directly using the crude reaction mixture obtained from the hydrogenator. Thus, a Teflon AF2400 tubing (1.5 mL volume) in the form of a "tube-in-tube" reactor (Uniqsis) was installed at the output of the H-Cube Pro. The advantage of using a tube-in-tube reactor is that the H₂ released from the inner to the outer tube can be readily removed by the ventilation system of the fume hood. HCl (37%) was introduced into the system after the tube-in-tube reactor with a standard T-mixer (20 µL min⁻¹, 1.2 equiv). The acidified solution entered a new residence time unit (0.8 mm inner diameter tubing) immersed in an oil bath. A back pressure regulator (BPR) was installed at the end of the reactor. The purpose of the BPR is to avoid boiling of the solvent during the cyclization if high temperatures are required, and to enhance removal of H₂ through the AF2400 material. Several BPR were tested, and complete removal of the H₂ excess within the 1.5 mL AF2400 tube was achieved when the pressure was increased to 18 bar.

Under these conditions full conversion of the 2,2-difluoro-2-(2,4-dinitro-5-fluoro-phenoxy)-N,N-dimethyl-acetamide to the 2,2-difluoro-2-(2,4-diamino-5-fluoro-phenoxy)-N,N-dimethyl-acetamide can be assured (cf. Table 1). A residence time of 10 min at 120 °C was initially tested for the cyclization step. At this temperature full conversion of the 2,2-difluoro-2-(2,4-diamino-5-fluoro-phenoxy)-N,N-dimethyl-acetamide intermediate was expected and possible side products for the reaction could be evaluated. As anticipated, HPLC and GCMS analyses of the crude reaction mixture collected from the output revealed complete conversion of the starting dinitro-FPAA (3) as well as the 2,2-difluoro-2-(2,4-diamino-5-fluoro-phenoxy)-N,N-dimethyl-acetamide intermediate. The desired 6-amino-2,2,7-trifluoro-4H-benzo-[1,4]-oxazin-3-one was formed with very good selectivity, although a small amount (1% according to GCMS) of hydrolysis product was also observed possibly due to the high temperature used in this process. Reaction temperature and residence time were gradually decreased (Table 2) to ultimately reveal 80 °C and 5 min residence time as optimal conditions for the cyclization. Under these conditions excellent product selectivity with high purity profiles in both HPLC and GCMS analyses were achieved. Further temperature decrease (entry 5, Table 2) reduced the conversion of the 2,2-difluoro-2-(2,4-diamino-5-fluoro-phenoxy)-N,N-dimethyl-acetamide intermediate.

**Table 2: Results obtained for the sequential hydrogenation/cyclization continuous flow process.**

| Entry | Temperature (°C) | Cyclization Residence Time (min) | Conversion to ABO (%)^{a} | Selectivity (%)^{a} |
|---|---|---|---|---|
| 1 | 120 | 10 | > 99 | 99 |
| 2 | 100 | 10 | > 99 | > 99 |
| 3 | 100 | 5 | > 99 | > 99 |
| 4 | 80 | 5 | > 99 | > 99 |
| 5 | 60 | 5 | 77 | > 99 |

| | | | | |
|---|---|---|---|---|
| ^{a} Evaluated by GCMS and HPLC peak integration area (215 nm). | | | | |

### Sequential Continuous Flow Nitration/Hydrogenation/Cyclization of 2,2-difluoro-2-(3-fluoro-phenoxy)-N,N-dimethyl-acetamide.

For the experiments described herein we assembled the two main reactor parts described above (i.e., dinitration/liquid-liquid extraction and hydrogenation/degassing/cyclization). For this purpose, the output of the liquid-liquid membrane separator was connected to a T-mixer where the stream was mixed with methanol, and then fed to the input pump of the H-Cube Pro.
All 6 feeds in the continuous flow setup were started, setting all reaction parameters as stated above. The system was let run for 60 min to assure stability and steady-state concentration conditions of the dinitration part of the setup. After ensuring that the initial dinitration/liquid-liquid extraction part of the setup was performing as expected, the inlet of the H-Cube Pro was switched from solvent to the reaction mixture (after the hydrogenation/degassing/cyclization part of the setup had been previously stabilized). After approximately 15 min the crude dark brown reaction mixture was collected in fractions of 15 min each, diluted with methanol to a known volume, and analyzed by quantitative HPLC.

After minor modifications in the setup (the residence time for the cyclization was increased from 5 to 10 min) the fully integrated continuous flow process to obtain 6-amino-2,2,7-trifluoro-4H-benzo-[1,4]-oxazin-3-one was carried out for more than 75 min providing excellent results. After ca. 100 min we could observe that 2,2-difluoro-2-(2,4-dinitro-5-fluoro-phenoxy)-N,N-dimethyl-acetamide was no longer fully hydrogenated to 2,2-difluoro-2-(2,4-diamino-5-fluoro-phenoxy)-N,N-dimethyl-acetamide, with 1-2% of the mononitro/monoamino-derivative being detected. ICPMS results. It should be noted that the CatCart catalyst cartridge only contains 275 mg of Pd/C, which corresponds to a catalyst loading for this amount of reaction mixture of ca. 1 mol% Pd. Quantitative HPLC analysis of the crude reaction mixture obtained during the initial 60 min revealed a product yield for the overall process of 83%. After the analysis a 3 M aqueous solution of NaOH was added dropwise to the crude reaction mixture until a pH of 4-5 was reached. The solvent was then evaporated under reduced pressure to ca. 20% of the initial volume and 150 mL of water were added. The precipitate formed was filtered off, washed with cold water, and dried at 50 °C under reduced pressure (2.28 g, 87% yield, 91 % pure by quantitative HPLC). Notably, the product yield obtained for the overall continuous flow process was significantly superior compared to the reported batch protocol (ca. 50%).

The continuous flow reactor depicted above was started, setting all parameters as stated. The thermostated baths and the column reactor from the H Cube Pro were preheated to the appropriate temperatures. The H Cube Pro pump was set to 1 mL min⁻¹ and the instrument to "full H₂ mode". Susequently, neat, 2,2-difluoro-2-(3-fluoro-phenoxy)-N,N-dimethyl-acetamide (35.8 µL min⁻¹) was pumped through the reactor. When the system had stabilized, the crude reaction was collected from the reactor output for 60 min. The obtained biphasic mixture was homogenized with methanol (40 mL) and the pH adjusted with 3 M NaOH to 4-5. The solvent was evaporated under reduced pressure to approximately 20% of the initial volume. After addition of water and stirring for 10 min the precipitate was filtered off, washed with water, and dried at 50 °C (2.28 g, 87%, 91% pure by quantitative HPLC); mp. 210-220 °C (dec.); ¹H NMR (300 MHz, DMSO) δ 11.71 (s, 1 H), 7.13 (d*, J* = 11.1 Hz, 1H), 6.51 (d, *J* = 8.6 Hz, 1H), 5.29 (s, 2H). ¹³C NMR (75 MHz, DMSO) δ 154.6, 154.1, 153.6, 148.0, 144.9, 134.7, 134.6, 127.6, 127.5, 121.6, 121.6, 117.2, 113.7, 110.2, 105.5, 105.2, 102.8.

### Conclusions

A continuous flow protocol for the preparation of 6-amino-2,2,7-trifluoro-4H-benzo-[1,4]-oxazin-3-one using a fully continuous, three-step sequential nitration/hydrogenation / cyclization process. The sequence starts from the 2,2-difluoro-2-(3-fluoro-phenoxy)-N,N-dimethyl-acetamide precursor, which is dinitrated using 100% HNO₃ in oleum. Control of the reaction exotherm and very efficient mixing has been achieved using a glass static mixer, thus ensuring constant conversion profiles and purity for the dinitro intermediate. The nitration mixture was then quenched with water and extracted with toluene at 0 °C. A membrane based liquid-liquid separator was used after the extraction process to separate the aqueous and organic phases. The first nitration step took place with excellent yield (92% isolated) and purity for the desired 2,2-difluoro-2-(2,4-dinitro-5-fluoro-phenoxy)-N,N-dimethyl-acetamide. The toluene solution of desired 2,2-difluoro-2-(2,4-dinitro-5-fluoro-phenoxy)-N,N-dimethyl-acetamide, after mixing with methanol, entered a continuous flow hydrogenator, where desired 2,2-difluoro-2-(2,4-dinitro-5-fluoro-phenoxy)-N,N-dimethyl-acetamide was reduced to 2,2-difluoro-2-(2,4-diamino-5-fluoro-phenoxy)-N,N-dimethyl-acetamide using heterogeneous Pd/C as catalyst at 45 °C. The reduced intermediate was then acidified inline with HCl 37% and cyclized to 6-amino-2,2,7-trifluoro-4H-benzo-[1,4]-oxazin-3-one at 80 °C for 10 min using an additional residence time unit.
The overall yield for the continuous flow process was 83% (quantitative HPLC) (87% isolated with 91% purity), improving significantly the yield obtained in the reported batch protocol for the three-step sequence.

## Claims

1. A process for manufacturing 1,4-benzoxazinones of formula (I), 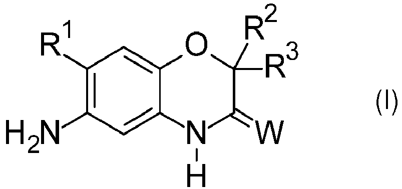 wherein
R¹ is H or halogen;
R² is halogen;
R³ is H or halogen; and
W is O or S
**characterized in that** in a fully integrated continuous process the following steps (a) to (c) are performed:
step (a): dinitrating the aryloxyacetamide of formula (IV) 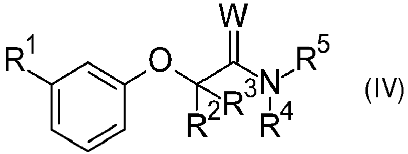 wherein
R¹ is H or halogen;
R2 is halogen;
R³ is H or halogen;
and
R⁴, R⁵ are independently of each other C₁-C₆-alkyl, C₂-C₆-alkenyl, C₂-C₆-alkynyl, C₁-C₆-haloalkyl, C₁-C₆-cyanoalkyl, C₁-C₆-nitroalkyl, C₁-C₆-hydroxyalkyl, C₁-C₆-alkoxy-C₁-C₆-alkyl, amino-C₁-C₆-alkyl, (C₁-C₆-alkyl)amino-C₁-C₆-alkyl, di(C₁-C₆-alkyl)amino-C₁-C₆-alkyl, C₃-C₆-cycloalkyl, phenyl or benzyl, wherein the phenyl and the benzyl ring are independently of one another unsubstituted or substituted with1 to 5 substituents selected from the group consisting of halogen, C₁-C₆-alkyl or C₁-C₆-alkoxy; or
R⁴ and R⁵ together with the N atom which they are attached to, represent a saturated or aromatic 3- to 6-membered ring, optionally containing 1 to 3 additional heteroatoms from the group O, S and N, with the ring optionally being substituted with 1 to 3 C₁-C₆-alkyl substituents; and W isOorS;
followed by step (b): reduction of the nitro compounds of formula (III) resulting from step (a): 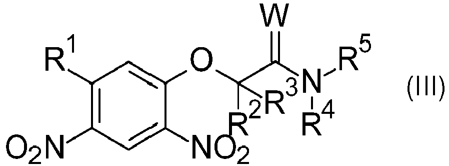 wherein R¹, R², R³, R⁴, R⁵ and W are defined as above;
followed by step (c): cyclization of the amino compounds of formula (II) resulting from step (b): 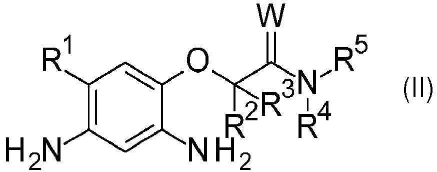 wherein R¹, R², R³, R⁴, R⁵ and W are defined as above;
under acidic conditions to give 1,4-benzoxazinones of formula (I).

2. The process according to claim 1, wherein R¹ and R³ are halogen and R² is F.

3. The process according to claims 1 or 2, wherein R⁴ and R⁵ independently of each other are C₁-C₆-alkyl.

4. The process according to any of claims 1 to 3, wherein the aryloxyacetamide of formula (IV) is subjected to an initial continuous flow dinitration.

5. The process according to any of claims 1 to 4, wherein in step (a) a microstructured device heated to 60 °C is used.

6. The process according to any of claims 1 to 5, wherein the nitro compound of formula (III) is separated from the reaction mixture with a continuous liquid-liquid membrane separator.

7. The process according to any of claims 1 to 6, wherein the organic phase containing the nitro compound of formula (III) resulting from step (a) is subjected to continuous hydrogenation, immediately consuming the nitro compound of formula (III).

8. The process according to any of claims 1 to 7, wherein step (b) is made over a Pd/C fixed bed catalyst.

9. The process according to any of claims 1 to 8, wherein step (c) is made in a tubular reactor.
